# EUROPEAN PATENT APPLICATION

(11) **EP 3 708 153 A1**
(43) Date of publication of application: **16.09.2020**
(21) Application number: 18876729.7
(22) Date of filing: 18.05.2018
(51) Int. Cl.: A61K 9/12, A61K 31/198, A61P 11/10

(54) **SOLUTION PREPARATION FOR AEROSOL INHALATION OF CARBOCISTEINE, AND PREPARATION METHOD THEREFOR**

(30) Priority: 08.11.2017 CN 201711089083
(71) Applicant: Beijing Increase Innovation Drug Research Co., Ltd, Beijing 102200 (CN)
(72) Inventor: ZHANG, Baoxian, Beijing 102200 (CN); HU, Jie, Beijing 102200 (CN)
(74) Representative: Seemann & Partner Patentanwälte mbB
(86) International application number: PCT/CN2018/087527
(87) International publication number: WO 2019/091082

(57) **Abstract**

The present disclosure provides a solution preparation for aerosol inhalation of carbocisteine, and a preparation method therefor. The solution preparation for aerosol inhalation of carbocisteine of the present disclosure comprises carbocisteine, a salt thereof and/or a hydrate thereof, a metal complexing agent, a pH adjusting agent, and water for injection. The solution preparation for aerosol inhalation of carbocisteine of the present disclosure has the characteristics of high efficiency, low toxicity, good stability and high safety, and particularly, greatly reduces the hepatotoxicity and the renal toxicity to patients using the drug.

## Description

### TECHNICAL FIELD

The present disclosure belongs to the technical field of pharmaceutical preparation, specifically relates to a solution preparation for aerosol inhalation of carbocisteine and a preparation method therefor.

### BACKGROUND

Due to factors such as the dense population, the large number of smoking people and environmental pollution, the morbidity and mortality of respiratory system diseases are high in recent years. According to the data of American statistical yearbook, among all categories of causes of death, the ranking of the death caused by respiratory tract-related diseases (excluding tumors) has increased from the 10th in 1970 to the 4th (chronic obstructive pulmonary disease) and the 8th (pneumonia, influenza and upper respiratory tract infection) in 1991, and the morbidity of respiratory system diseases in China ranks first among various diseases in any age group. This problem has increasingly aroused people's attention, and therefore the development of drugs for treating respiratory system diseases (simply referred to as respiratory drugs) has also become an important research and development field of drug research.

Cough and expectoration are common symptoms of respiratory system diseases in clinic. Excessively frequent and severe cough not only increases pain of patients, influences rest and sleep, increases consumption of physical strength, but also even promotes the progression of diseases, and causes other complications, such as pneumonia, chronic pharyngitis, chronic bronchitis, bronchiectasis, pulmonary abscess, cavitary pulmonary tuberculosis, and the like. In such cases, in addition to the symptomatic treatment aiming at the respiratory system diseases, antitussive and expectorant drugs are also needed to be properly applied to relieve cough. The application of antitussive drug(s) is only to relieve symptoms, and fundamentally, the treatment should aim at the etiology of cough. Most of the cough is caused by inflammatory mediators, for example, the mediators excessively released in diseases such as tracheitis, asthma, pneumonia, lung tumors, and the like, therefore, expectorants are further needed to be used according to the state of a disease.

Currently, most of the commonly used expectorants are compound preparations of chemical drugs containing the central antitussive codeine or dextromethorphan hydrobromide and the expectorant guaiacol glyceryl ether as effective ingredients. The effect of dextromethorphan is similar to or stronger than codeine, and in June 2005, the U.S. FDA issued an announcement, indicating that improper use of dextromethorphan might cause death or other adverse effects, such as brain damage, loss of consciousness, and arrhythmia.

In recent years, carbocisteine is used for treating diseases such as sputum thickening, difficulty in expectoration and phlegm obstruction in trachea caused by diseases such as chronic bronchitis and bronchial asthma. Carbocisteine mainly acts on the secretion of bronchial glands, increases the secretion of low-viscosity salivary mucin, reduces the production of high-viscosity fucomucin, thereby reducing the viscosity of the sputum to facilitate expectoration. Patent document 1 discloses an oral solution of carbocisteine and a preparation method thereof, and the oral solution of carbocisteine is used for treating sputum thickening or difficulty in expectoration caused by diseases such as chronic bronchitis and bronchial asthma. Said oral solution contains 2% to 10% of carbocisteine, sodium hydroxide, an antioxidant, a combination of solution stabilizers and a flavoring agent, and the pH value of the oral solution is 6.0 to 7.5. The above oral solution of carbocisteine is prepared by subjecting carbocisteine to salt formation with a sodium hydroxide solution, dissolving the resultant in water, adding the antioxidant, the combination of solution stabilizers and the flavoring agent, stirring, diluting to volume, filtering, bottling, and sterilizing. However, the adverse effects such as a large administered dosage of the oral solution, much hepatic and renal metabolism, a tendency of easily causing damage to organ(s), a relatively small amount of drug reaching the target organ(s), a slow onset of action, and the like are present in Patent document 1.

### PRIOR ART LITERATURE

### PATENT LITERATURE

Patent document 1: CN 104511025A

### SUMMARY

### Technical problem

In view of the above, the technical problem to be solved by the present disclosure is to provide a solution preparation for aerosol inhalation of carbocisteine, which is used for treating diseases such as sputum thickening, difficulty in expectoration and phlegm obstruction in trachea caused by chronic bronchitis, bronchial asthma and the like. The solution preparation for aerosol inhalation of carbocisteine of the present disclosure is directly inhaled from mouth and nose, thereby avoiding the first-pass effect in liver and the damage and degradation in gastrointestinal tract, greatly reducing the metabolic process of the drug by liver and kidney and significantly reducing the damage to the organ(s) of a patient.

The solution preparation for aerosol inhalation of carbocisteine of the present disclosure makes up for the blank in the current domestic market and provides a novel, safe and effective drug preparation of carbocisteine and an administration mode.

### Solution to the problems

The present disclosure provides a solution preparation for aerosol inhalation of carbocisteine comprising: carbocisteine, a salt thereof and/or a hydrate thereof; a metal complexing agent; a pH adjusting agent; and water for injection.

In a preferred embodiment, the preparation also comprises one or more pharmaceutical excipients suitable for pulmonary administration, and the pharmaceutical excipient includes an antioxidant and a surfactant.

In a preferred embodiment, a single dose of the preparation comprises: 20 to 120 mg of carbocisteine, a salt thereof and/or a hydrate thereof, calculated as free carbocisteine; 0.1 to 5 mg of the metal complexing agent; a suitable amount of the pH adjusting agent; and water for injection.

In a preferred embodiment, a single dose of the preparation comprises: 50 to 100 mg of carbocisteine, a salt thereof and/or a hydrate thereof, calculated as free carbocisteine; 0.5 to 2 mg of the metal complexing agent; a suitable amount of the pH adjusting agent; and water for injection.

In a preferred embodiment, the pH adjusting agent is sodium bicarbonate, sodium hydroxide, disodium hydrogen phosphate, sodium citrate, or dipotassium hydrogen phosphate.

In a preferred embodiment, the preparation has a pH value of 5 to 8.

In a preferred embodiment, the preparation has a pH value of 6 to 7.

In a preferred embodiment, the metal complexing agent comprises at least one selected from the group consisting of edetic acid, disodium edetate, and calcium disodium edetate.

In a preferred embodiment, the solution preparation for aerosol inhalation of carbocisteine is used for treating the following diseases: sputum thickening, difficulty in expectoration and phlegm obstruction in trachea caused by chronic bronchitis and bronchial asthma.

The present disclosure also provides a preparation method of the above solution preparation for aerosol inhalation of carbocisteine comprising the following steps:
(1) adding 50% to 80% of the total amount of water for injection into a liquid preparation apparatus, controlling the water temperature at 25 ± 10°C, filling nitrogen into the water for injection for protection until a liquid preparation is finished, keeping a positive nitrogen pressure in the liquid preparation apparatus, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding carbocisteine, a salt thereof and/or a hydrate thereof, and stirring until complete dissolution;
(4) adding a pH adjusting agent to adjust pH, simultaneously measuring the residual oxygen content until the residual oxygen content is less than 2 mg/L, and carrying out the next operation;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, and a filling volume is 5 mL.

### Advantageous effects

Compared with the prior art, the advantageous effects of the present disclosure are as follows.
(1) Compared with other dosage forms, the drug directly reaches the target organ(s), thereby avoiding the first-pass effect in liver and the damage and degradation in gastrointestinal tract and reducing the damage to the organs of the whole body, especially the damage to liver and kidney, and is safer to use.
(2) Since the solution preparation for aerosol inhalation of carbocisteine of the present disclosure is an inhalation-type preparation, it has a faster effect than an oral dosage form for respiratory system diseases, and avoids the defect that the drug needs to be absorbed by gastrointestinal tract first and then exerts systemic effect along with blood circulation and thus showing a slow onset of action.
(3) The stability of carbocisteine is improved, various detection indexes do not significantly change after the preparation is left to stand for a long period of time, and the product quality is ensured as qualified within the validity period.
(4) The added excipients are few in variety, the safety of administration is high, the production process is simple, the cost is low, and the industrial scale production may be realized.

### DETAILED DESCRIPTION

Various exemplary examples, features and aspects of the present disclosure will be described in detail below.

In addition, numerous specific details are set forth in the specific embodiments below in order to better illustrate the present disclosure. It should be understood by those skilled in the art that the present disclosure may also be implemented without some of these specific details. The following examples are merely for illustrating the present disclosure and should not be construed as limiting the scope of the present disclosure. Any equivalent replacement in the art made in accordance with the content of the present disclosure is within the protection scope of the present disclosure.

### <Solution preparation for aerosol inhalation of carbocisteine>

The solution preparation for aerosol inhalation of carbocisteine of the present disclosure comprises: carbocisteine, a salt thereof and/or a hydrate thereof; a metal complexing agent; a pH adjusting agent; and water for injection.

By using the solution preparation for aerosol inhalation of carbocisteine of the present disclosure, diseases such as sputum thickening, difficulty in expectoration and phlegm obstruction in trachea caused by chronic bronchitis, bronchial asthma and the like are able to be treated. In addition, the solution preparation for aerosol inhalation of carbocisteine of the present disclosure is directly inhaled from mouth and nose, thereby avoiding the first-pass effect in liver and the damage and degradation in gastrointestinal tract, greatly reducing the metabolic process of the drug by liver and kidney and significantly reducing the damage to the organ(s) of a patient. Also, it has a faster effect than an oral dosage form for respiratory system diseases, and avoids the defect that the drug needs to be absorbed by gastrointestinal tract first and then exerts systemic effect along with blood circulation and thus showing a slow onset of action.

The solution preparation for aerosol inhalation of carbocisteine of the present disclosure adopts the single-dose packaging for the drug. A single dose refers to the dose of the pharmaceutically active ingredient administered in a single inhalation. In the present specification, a single dose represents 5 mL.

The solution preparation for aerosol inhalation of carbocisteine provided by the present disclosure is a single-dose preparation. The preparation is convenient to use, does not need to be diluted and formulated, may greatly reduce microbial pollution and waste in the using process, and avoids the defect caused by a multi-dose large-package solution, i.e., repeated measuring and repeated dilution and formulation are easy to cause the breeding of microbes.

The present disclosure provides a novel preparation which is lack in the prior art and has accurate drug dosage, superior and stable drug quality, and safe and simple clinical application, and a preparation method thereof.

Hereinafter, each ingredient constituting the solution preparation for aerosol inhalation of carbocisteine of the present disclosure will be described.

### [Carbocisteine, a salt thereof and/or a hydrate thereof]

In a single dose of the solution preparation for aerosol inhalation of carbocisteine of the present embodiment, calculated as free carbocisteine, preferably, 20 to 120 mg of carbocisteine, a salt thereof and/or a hydrate thereof is contained, and more preferably, 50 to 100 mg of carbocisteine, a salt thereof and/or a hydrate thereof is contained.

When the content of carbocisteine, a salt thereof and/or a hydrate thereof in a single dose of the solution preparation for aerosol inhalation of carbocisteine of the present disclosure is within the above range, the viscosity of sputum is able to be reduced, and said solution preparation is favorably used for treating diseases such as sputum thickening, difficulty in expectoration and phlegm obstruction in trachea caused by chronic bronchitis, bronchial asthma and the like.

### [Metal complexing agent]

The metal complexing agent contained in the solution preparation for aerosol inhalation of carbocisteine of the present disclosure has complexing ability, and the stability of the preparation may be improved.

As examples of the metal complexing agent, for example, edetic acid, edetate such as disodium edetate and calcium disodium edetate, and the like may be exemplified. Among these, edetate is preferred, and disodium edetate is more preferred. These metal complexing agents have relatively strong ability to complex with metal ion(s) and the complexed ions have a plurality of types, especially edetate, so that the influence of the metal ions introduced in the liquid preparation process on the quality of the drug solution may be avoided. These metal complexing agents may be used alone, or may be used in combination of two or more.

In a single dose of the solution preparation for aerosol inhalation of carbocisteine, preferably, 0.1 to 5 mg of a metal complexing agent is contained, and more preferably, 0.5 to 2 mg of a metal complexing agent is contained. By containing the metal complexing agent in the above range, it is able to facilitate the exertion of the efficacy of the solution preparation for aerosol inhalation of carbocisteine and improve the stability of the solution preparation for aerosol inhalation of carbocisteine. Various detection indexes do not significantly change even after the preparation is left to stand for a long period of time, and the product quality is ensured as qualified in the validity period.

If the content of the metal complexing agent is less than 0.1 mg, the stability of carbocisteine becomes poor, various detection indexes change significantly after the preparation is left to stand for a long period of time, and the product quality is reduced. If the content of the metal complexing agent is greater than 5 mg, the ingested metal complexing agent such as disodium edetate may form a water-soluble chelate with the calcium ions in a human body and be excreted out of the body, and excessive intake of metal complexing agent may cause hypocalcemia or loss of bone calcium. Therefore, the content of the metal complexing agent such as disodium edetate in the product should be reduced as far as possible on the premise of ensuring effective antioxidation.

### [pH adjusting agent]

The solution preparation for aerosol inhalation of carbocisteine of the present disclosure contains a pH adjusting agent in order to adjust the pH value.

As a pH adjusting agent, it is not particularly limited as long as the above effects of the solution preparation for aerosol inhalation of carbocisteine is not impaired, for example, sodium bicarbonate, sodium hydroxide, disodium hydrogen phosphate, sodium citrate, dipotassium hydrogen phosphate, sodium carbonate, sodium dihydrogen phosphate, hydrochloric acid, sulfuric acid, lactic acid, malic acid, acetic acid, phosphoric acid, citric acid and the like may be exemplified. These pH adjusting agents may be used alone, or may be used in combination of two or more. Among these, sodium bicarbonate, sodium hydroxide, disodium hydrogen phosphate, sodium citrate, and dipotassium hydrogen phosphate are more preferred.

The content of the pH adjusting agent may be determined appropriately according to the content of other ingredients, so that the pH value of the solution preparation for aerosol inhalation of carbocisteine is adjusted to a target value. In a single dose of the solution preparation for aerosol inhalation of carbocisteine of the present disclosure, the content of the pH adjusting agent is preferably 10.8 to 12.4 mg/mL, and more preferably 11.2 to 11.4 mg/mL. By containing the pH adjusting agent in the above range, it is able to favorably facilitate the salt formation of carbocisteine and increase the solubility of carbocisteine.

The pH value of the solution preparation for aerosol inhalation of carbocisteine of the present disclosure is preferably 5 to 8, and more preferably 6 to 7. By setting the range of the pH value of the solution preparation for aerosol inhalation of carbocisteine as the above range, the solubility of carbocisteine may be increased. If the pH value is less than 5, an adverse effect of incomplete dissolution of the carbocisteine raw material in water may be generated. On the other hand, if the pH value exceeds 8, there is a tendency that the color of the solution turns yellow and the amount of impurities increases, which is not preferred.

### [Pharmaceutical excipient]

The solution preparation for aerosol inhalation of carbocisteine of the present disclosure may contain one or more pharmaceutical excipients suitable for pulmonary administration as required.

The pharmaceutical excipient is not particularly limited, and examples thereof include an antioxidant, a surfactant, a flavoring agent, a stabilizer, and the like. Preferably, the pharmaceutical excipient includes an antioxidant and a surfactant. As an antioxidant, for example, sodium metabisulfite, sodium sulfite, sodium bisulfite, sodium thiosulfate, acetylcysteine and the like may be preferably exemplified. As a surfactant, for example, glycerin fatty acid ester, sorbitan fatty acid ester, polyoxyethylene fatty acid ester, polyethylene glycol fatty acid ester, sucrose fatty acid ester, and the like may be preferably exemplified.

### <Preparation method of the solution preparation for aerosol inhalation of carbocisteine>

In the present disclosure, the preparation steps of the solution preparation for aerosol inhalation of carbocisteine are not particularly limited, in principle, any method for preparing the preparation by mixing carbocisteine, a salt thereof and/or a hydrate thereof, a metal complexing agent, a pH adjusting agent and water for injection is suitable for the present disclosure. The preparation method of the preparation of the present disclosure is preferably as follows:
(1) adding 50% to 80% of the total amount of water for injection into a liquid preparation apparatus, controlling the water temperature at 25 ± 10°C, filling nitrogen into the water for injection for protection until a liquid preparation is finished, keeping a positive nitrogen pressure in the liquid preparation apparatus, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding carbocisteine, a salt thereof and/or a hydrate thereof, and stirring until complete dissolution;
(4) adding a pH adjusting agent to adjust pH, simultaneously measuring the residual oxygen content until the residual oxygen content is less than 2 mg/L, and carrying out the next operation;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, and a filling volume is 5 mL.

### EXAMPLES

The present disclosure will be described in detail below with reference to Examples, but the protection scope of the present disclosure is not limited thereto.

Prescription 1: single-dose solution preparation for aerosol inhalation of carbocisteine

| | |
|---|---|
| carbocisteine | 20 mg |
| disodium edetate | 0.1 mg |
| sodium hydroxide | suitable amount, pH adjusted to 5 |

Prescription 2: single-dose solution preparation for aerosol inhalation of carbocisteine

| | |
|---|---|
| carbocisteine | 40 mg |
| disodium edetate | 0.5 mg |
| sodium hydroxide | suitable amount, pH adjusted to 6 |

Prescription 3: single-dose solution preparation for aerosol inhalation of carbocisteine

| | |
|---|---|
| carbocisteine | 50 mg |
| disodium edetate | 2 mg |
| sodium hydroxide | suitable amount, pH adjusted to 6 |

Prescription 4: single-dose solution preparation for aerosol inhalation of carbocisteine

| | |
|---|---|
| carbocisteine | 80 mg |
| disodium edetate | 5 mg |
| sodium hydroxide | suitable amount, pH adjusted to 7 |

Prescription 5: single-dose solution preparation for aerosol inhalation of carbocisteine

| | |
|---|---|
| carbocisteine | 100 mg |
| disodium edetate | 1.25 mg |
| sodium hydroxide | suitable amount, pH adjusted to 8 |

Prescription 6: single-dose solution preparation for aerosol inhalation of carbocisteine

| | |
|---|---|
| carbocisteine | 120 mg |
| disodium edetate | 4 mg |
| sodium hydroxide | suitable amount, pH adjusted to 7 |

Prescription 7: single-dose solution preparation for aerosol inhalation of carbocisteine

| | |
|---|---|
| carbocisteine | 120 mg |
| edetic acid | 3 mg |
| sodium bicarbonate | suitable amount, pH adjusted to 7.5 |

Prescription 8: single-dose solution preparation for aerosol inhalation of carbocisteine

| | |
|---|---|
| carbocisteine | 100 mg |
| edetic acid | 1.25 mg |
| sodium hydroxide | suitable amount, pH adjusted to 8 |

Prescription 9: single-dose solution preparation for aerosol inhalation of carbocisteine

| | |
|---|---|
| carbocisteine | 100 mg |
| calcium disodium edetate | 1.25 mg |
| sodium hydroxide | suitable amount, pH adjusted to 8 |

Prescription 10: single-dose solution preparation for aerosol inhalation of carbocisteine

| | |
|---|---|
| carbocisteine | 100 mg |
| disodium edetate | 0.5 mg |
| sodium hydroxide | suitable amount, pH adjusted to 8 |

Prescription 11: single-dose solution preparation for aerosol inhalation of carbocisteine

| | |
|---|---|
| carbocisteine | 100 mg |
| disodium edetate | 1 mg |
| sodium hydroxide | suitable amount, pH adjusted to 8 |

Prescription 12: single-dose solution preparation for aerosol inhalation of carbocisteine

| | |
|---|---|
| carbocisteine | 100 mg |
| disodium edetate | 2 mg |
| sodium hydroxide | suitable amount, pH adjusted to 8 |

Prescription 13: single-dose solution preparation for aerosol inhalation of carbocisteine

| | |
|---|---|
| carbocisteine | 100 mg |
| disodium edetate | 4 mg |
| sodium hydroxide | suitable amount, pH adjusted to 8 |

Prescription 14: single-dose solution preparation for aerosol inhalation of carbocisteine

| | |
|---|---|
| carbocisteine | 100 mg |
| sodium hydroxide | suitable amount, pH adjusted to 8 |

Example 1: 1000 mL of a solution preparation for aerosol inhalation of carbocisteine was prepared according to the proportion of Prescription 1.

70% of the total amount of water for injection, which was 700 mL, was added into a liquid preparation apparatus, the water temperature was controlled at 30°C, nitrogen was filled into the water for injection for protection until the liquid preparation was finished, a positive nitrogen pressure was kept in the liquid preparation apparatus, and the next operation was carried out after the residual oxygen content was measured to be 1.6 mg/L.

Then, 20 mg of disodium edetate (manufactured by Chengdu Huayi Pharmaceutical Excipient Manufacturing Co., Ltd.) was weighed and slowly added into the above water for injection, and the mixture was stirred until the disodium edetate was completely dissolved.

Next, 4 g of carbocisteine (manufactured by Sinopharm Shantou Jinshi Pharmaceutical Co., Ltd.) was slowly added, and the mixture was stirred until carbocisteine was completely dissolved.

Then, a suitable amount of sodium hydroxide was added to adjust pH to 5, the residual oxygen content was simultaneously measured until it was 1.5 mg/L, and the next operation was carried out.

Next, water for injection was further added to full amount (1000 mL) and the mixture was stirred for 5 minutes to mix uniformly.

Then, the resultant was filtered with a 0.45-µm filter membrane for primary filtration and filtered with a 0.22-µm filter membrane for fine filtration, both filtrations were sterile filtrations. The resultant was bottled and encapsulated in an ampoule and filled the ampoule with nitrogen gas, and the filling volume was 5 mL.

### Examples 2 to 13

A solution preparation for aerosol inhalation of carbocisteine was prepared in the same manner as in Example 1, except that the proportions of Prescriptions 2 to 13 were used in place of that of Prescription 1.

### Comparative example 1

A solution preparation for aerosol inhalation of carbocisteine was prepared in the same manner as in Example 1, except that the proportion of Prescription 14 was used in place of that of Prescription 1.

In order to further illustrate the technical effects of the present disclosure, the following specific experimental examples were provided.

### Experimental example 1

The single-dose solution preparations for aerosol inhalation of carbocisteine obtained in Examples 5, 8 and 9 (the solution preparations for aerosol inhalation of carbocisteine were prepared according to the formulas of Prescriptions 5, 8 and 9) were left to stand for one year. The content of carbocisteine in the single-dose solution preparations for aerosol inhalation of carbocisteine obtained in Examples 5, 8 and 9 were determined by the following method for determining the content of carbocisteine after one year, and the color and clarity of the preparations were observed by visual observation. The results were shown in Table 1.

Determination of the content of carbocisteine
(1) Octadecylsilane-bonded silica gel was used as a filler (4.6 × 150 mm, 5 µm), a 20 mM sodium octane sulfonate solution and a 20 mM potassium dihydrogen phosphate solution (pH was adjusted to 2.5 with phosphoric acid) were used as the mobile phase, the detection wavelength was 210 nm, the column temperature was 30°C, and the flow rate was 1.0 mL/min.
(2) An appropriate amount of the above test sample was precisely measured, a solution containing 0.25 mg of carbocisteine per 1 mL was prepared by quantitatively diluting the test sample with the mobile phase, and the solution was used as a sample solution to be tested; in addition, an appropriate amount of carbocisteine reference substance was precisely weighed, the mobile phase was added to dissolve and quantitatively dilute the carbocisteine reference substance to prepare a solution containing 0.25 mg of carbocisteine per 1 mL as a reference solution. 20 µL of the sample solution to be tested and 20 µL of the reference solution were each precisely measured and respectively injected into a liquid chromatograph, and the chromatograms were recorded. The content of carbocisteine was obtained by calculating based on the peak area according to the external standard method.

**Table 1**

| | Metal complexing agent | Color and clarity of the preparation | Content of carbocisteine (%) |
|---|---|---|---|
| Example 5 | disodium edetate | colorless, clear | 98.4 |
| Example 8 | edetic acid | slightly yellow, clear | 93.2 |
| Example 9 | calcium disodium edetate | slightly yellow, slightly turbid | 97.1 |

As could be seen from Table 1, even after the solution preparation for aerosol inhalation of carbocisteine of the present disclosure was left to stand for one year, the content of carbocisteine was still relatively high, the stability of carbocisteine was excellent, and the change in color and clarity of the preparation was small.

### Experimental example 2

The single-dose solution preparations for aerosol inhalation of carbocisteine obtained in Examples 10 to 13 and Comparative example 1 (the solution preparations for aerosol inhalation of carbocisteine were prepared according to the formulas of Prescriptions 10 to 14) were left to stand for one year. The content of carbocisteine in the single-dose solution preparations for aerosol inhalation of carbocisteine obtained in Examples 10 to 13 and Comparative example 1 were determined by the above method for determining the content of carbocisteine after one year, and the color and clarity of the preparations were observed by visual observation. The results were shown in Table 2.

**Table 2**

| | Content of disodium edetate in single-dose solution preparation for aerosol inhalation of carbocisteine (mg) | Color and clarity of the preparation | Content of carbocisteine (%) |
|---|---|---|---|
| Example 10 | 0.5 | slightly pale yellow, clear | 96.4 |
| Example 11 | 1 | colorless, clear | 95.9 |
| Example 12 | 2 | colorless, clear | 94.3 |
| Example 13 | 4 | colorless, clear | 94.4 |
| Comparative example 1 | 0 | pale yellow, slightly turbid | 89.2 |

As could be seen from Table 2, even after the solution preparation for aerosol inhalation of carbocisteine of the present disclosure was left to stand for one year, the content of carbocisteine was still relatively high, the stability of carbocisteine was excellent, and the change in color and clarity of the preparation was small.

However, in Comparative example 1 without disodium edetate, after the preparation was left to stand for one year, the content of carbocisteine was significantly reduced, the color of the preparation was significantly changed, and a phenomenon of slight turbidity also appeared.

### Experimental example 3

The comparison of respective monitoring data of hepatocytes of the solution preparation for aerosol inhalation of carbocisteine of the present disclosure (prepared according to Example 5) and a commercially available carbocisteine oral solution (trade name: BRONCHOKOD, dosage form: oral solution, prescription composition: carbocisteine, saccharin sodium, methylparaben, sodium hydroxyethyl cellulose, essence, sodium hydroxide and purified water, manufactured by A NATTERMANN & CIE. GMBH) at the time of administration to mice was carried out.

60 mice (20 ± 2 g) with half male and half female were selected and randomly divided into 3 groups, namely, a normal control group, an oral administration group and an aerosol inhalation administration group, with 20 mice in each group. The solution preparation for aerosol inhalation of carbocisteine prepared according to Example 5 was provided in the aerosol inhalation administration group, the same amount of the above commercially available carbocisteine oral solution was used for intragastric administration in the oral administration group, and the same amount of normal saline was administered to the mice in the normal control group by intraperitoneal injection. The administration lasted for two weeks, wherein the administration was respectively carried out once at 10 am and 4 pm every day with a dose of 0.1 mL each time. After two weeks of administration, the values of aspartate aminotransferase (AST), alkaline phosphatase (ALP) and alanine aminotransferase (ALT) in the hepatocyte supernatants of the mouse models in each administration group were detected, and the effect of carbocisteine on inducing hepatic injury was studied by statistical analysis. The results were shown in Table 3.

**Table 3**

| Group | ALP (U/L) | ALT (U/L) | AST (U/L) |
|---|---|---|---|
| normal control group | 320.67 ± 39.54 | 27.2 ± 2.16 | 119.27 ± 10.44 |
| aerosol inhalation administration group | 346.51 ± 27.84 | 28.8 ± 2.71 | 128.92 ± 13.41 |
| oral administration group | 398.13 ± 40.64 | 30.16 ± 1.0 | 149.47 ± 21.90 |

As could be seen from Table 3, the values of ALP, ALT and AST of the mice in the oral administration group were significantly increased (P < 0.01), while the ALP, ALT and AST activities of the mice in the aerosol inhalation administration group were significantly lower (P < 0.01) than those of the mice in the oral administration group. In the aerosol inhalation administration group, the effect of inducing hepatic injury was small and the drug administration was safer.

The above are only specific embodiments of the present disclosure, but the protection scope of the present disclosure is not limited thereto, and any changes or substitutions that may be easily conceived by those skilled in the art within the technical scope revealed by the present disclosure should be encompassed within the protection scope of the present disclosure. Therefore, the protection scope of the present disclosure should be subject to the protection scope of the claims.

## Claims

1. A solution preparation for aerosol inhalation of carbocisteine comprising: carbocisteine, a salt thereof and/or a hydrate thereof; a metal complexing agent; a pH adjusting agent; and water for injection.

2. The preparation according to claim 1, wherein the preparation also comprises one or more pharmaceutical excipients suitable for pulmonary administration and the pharmaceutical excipient comprises an antioxidant and a surfactant.

3. The preparation according to claim 1 or 2, wherein a single dose of the preparation comprises: 20 to 120 mg of carbocisteine, a salt thereof and/or a hydrate thereof, calculated as free carbocisteine; 0.1 to 5 mg of the metal complexing agent; a suitable amount of the pH adjusting agent; and water for injection.

4. The preparation according to any one of claims 1 to 3, wherein a single dose of the preparation comprises: 50 to 100 mg of carbocisteine, a salt thereof and/or a hydrate thereof, calculated as free carbocisteine; 0.5 to 2 mg of the metal complexing agent; a suitable amount of the pH adjusting agent; and water for injection.

5. The preparation according to any one of claims 1 to 4, wherein the pH adjusting agent is sodium bicarbonate, sodium hydroxide, disodium hydrogen phosphate, sodium citrate, or dipotassium hydrogen phosphate.

6. The preparation according to any one of claims 1 to 5, wherein the preparation has a pH value of 5 to 8.

7. The preparation according to claim 6, wherein the preparation has a pH value of 6 to 7.

8. The preparation according to any one of claims 1 to 7, wherein the metal complexing agent comprises at least one selected from the group consisting of edetic acid, disodium edetate, and calcium disodium edetate.

9. The preparation according to any one of claims 1 to 8, wherein the preparation is used for treating the following diseases: sputum thickening, difficulty in expectoration and phlegm obstruction in trachea caused by chronic bronchitis and bronchial asthma.

10. A preparation method of the preparation according to any one of claims 1 to 9 comprising the following steps:
(1) adding 50% to 80% of the total amount of water for injection into a liquid preparation apparatus, controlling a water temperature at 25 ± 10°C, filling nitrogen into the water for injection for protection until a liquid preparation is finished, keeping a positive nitrogen pressure in the liquid preparation apparatus, and carrying out the next operation after a residual oxygen content is measured to be less than 2 mg/L;
(2) weighing the metal complexing agent, slowly adding the metal complexing agent into the above water for injection, and stirring until the metal complexing agent is completely dissolved;
(3) slowly adding carbocisteine, a salt thereof and/or a hydrate thereof, and stirring until complete dissolution;
(4) adding a pH adjusting agent to adjust pH, simultaneously measuring the residual oxygen content until the residual oxygen content is less than 2 mg/L, and carrying out the next operation;
(5) further adding the rest of the total amount of water for injection and stirring to mix uniformly; and
(6) using a 0.45-µm filter membrane for a primary filtration and a 0.22-µm filter membrane for a fine filtration, both filtrations being sterile filtrations, bottling and encapsulating the resultant in an ampoule and filling the ampoule with nitrogen gas, and a filling volume is 5 mL.
